# EUROPEAN PATENT APPLICATION

(11) **EP 4 029 507 A1**
(43) Date of publication of application: **20.07.2022**
(21) Application number: 21152241.2
(22) Date of filing: 19.01.2021
(51) Int. Cl.: A61K 31/7004, A61K 33/14

(54) **PERITONEAL DIALYSIS SOLUTION FOR HYPERTENSIVE SUBJECTS**

(71) Applicant: Iperboreal Pharma Srl, 65122 Pescara (IT)
(72) Inventor: ARDUINI, Arduino, 6822 Arogno (CH)
(74) Representative: Cantaluppi, Stefano

(57) **Abstract**

The present invention refers to solutions for peritoneal dialysis for use in the treatment of end-stage renal disease in subjects with hypertension. Such solutions for peritoneal dialysis comprise from 1.36 to 4.5% w/v of glucose and 130 mmol/I of sodium and optional further ingredients.

## Description

### FIELD OF THE INVENTION

The present invention relates to the field of peritoneal dialysis.

In particular, it relates to solutions for peritoneal dialysis for use in subjects with hypertension.

### BACKGROUND OF THE INVENTION

Peritoneal dialysis (PD) is a viable but under-prescribed method of treating uremic patients (International comparison of peritoneal dialysis prescriptions from the Peritoneal Dialysis Outcomes and Practice Patterns Study (PDOPPS). Wang AY, Zhao J, Bieber B, Kanjanabuch T, Wilkie M, Marshall MR, Kawanishi H, Perl J, Davies S; PDOPPS Dialysis Prescription and Fluid Management Working Group. Perit Dial Int. 2020 Jan 17).

In peritoneal dialysis, a dialysis solution is introduced into the peritoneal cavity using a catheter achieving an exchange of solutes between the dialysate and the blood thanks to an osmotic gradient from the blood to the dialysate. To achieve and maintain a sufficient osmotic gradient a suitable amount of osmotic agent should be used. Glucose is a commonly used osmotic agent.

Hypervolemia is common in peritoneal dialysis and contributes to hypertension and left ventricular hypertrophy thus increasing the cardiovascular risk of these patients. The achievement of adequate water and sodium balance is considered a major determinant of dialysis adequacy (Silvio Borrelli, Vincenzo La Milia, Luca De Nicola, Gianfranca Cabiddu, Roberto Russo, Michele Provenzano, Roberto Minutolo, Giuseppe Conte, Carlo Garofalo, Study group Peritoneal Dialysis of Italian Society of Nephrology, Sodium removal by peritoneal dialysis: a systematic review and meta-analysis; J Nephrol 2019 Apr;32(2):231-239). Indeed, as volume retention is driven by salt retention, maintenance of salt balance should be a major goal since sodium and volume status play a key role in blood pressure regulation (Guyton AC. Dominant role of the kidneys and accessory role of whole-body autoregulation in the pathogenesis of hypertension. Am J Hypertens 1989; 2:575-85).

Sodium removal by peritoneal dialysis through the peritoneal membrane can occur by convection and by diffusion, whereby the former is more efficient than the latter (Van Biesen W, Williams JD, Covic AC, Fan S, Claes K, Lichodziejewska-Niemierko M, et al. Fluid status in peritoneal dialysis patients: the European body composition monitoring (EUROBCM) study cohort. PLOS One 2011; 6:e17148.). The extent of diffusive clearance is strongly dependent on the difference between dialysate and plasma-sodium concentrations. This difference is too small when standard PD solutions with a sodium concentration of 132 - 134 mmol/L are instilled in the peritoneal cavity. As a result, fluid overload and hypertension is mirrored in data from epidemiological studies, quantifying the proportion of hypertensive peritoneal dialysis patients or patients on anti-hypertensive medication at more than 80% (Cocchi R, Degli Esposti E, Fabbri A, Lucatello A, Sturani A, Quarello F, et al. Prevalence of hypertension in patients on peritoneal dialysis: results of an Italian multicentre study. Nephrol Dial Transplant 1999; 14:1536-40. Li W, Xu R, Wang Y, et al. Association of body mass index and uncontrolled blood pressure with cardiovascular mortality in peritoneal dialysis patients. J Human Hypert 2019; 33(2): 106-114. Shen JI, Mitani AA, Saxena AB, et al. Determinants of peritoneal dialysis technique failure in incident US patients. Perit Dial Int 2013; 33(2): 155-166.).

In view of the above, it is clear that correcting sodium overload and restoring sodium balance is a primary aim of long-term dialysis therapy (Peter G Blake, Sodium Levels in Peritoneal Dialysis Solution: How Low Should We Go?, Editorial Am J Kidney Dis. 2016 May;67(5):719-21).

Typical peritoneal dialysis solutions contain sodium in a concentration between 132 and 134 mmol/l.

The option of using low-sodium dialysate in hypertensive patients receiving peritoneal dialysis has been investigated. Since ultrafiltration is driven by osmotic pressure if dialysate sodium concentration is reduced without other alterations the consequent decline in solution osmolarity may lead to less ultrafiltration and thus offset the benefit. On the contrary, if glucose concentrations are increased to compensate the lower sodium concentration there might be undesirable metabolic consequences. Also, the use of hypertonic glucose solutions is often associated with peritoneal and systemic cardiovascular adverse effects. Furthermore, very low-sodium peritoneal dialysis solutions may lead to hyponatremia, volume depletion and hypotension (Blake, AJKD, 2016).

Clinical studies with low-sodium peritoneal dialysis solutions have been carried out. Sodium concentrations of 115, 102, 121 and 125 mEq/l were tested, compensated or uncompensated with higher glucose, eventually in combination with other osmotic agents, and with different daily regimens (Blake, AJKD, 2016).

However, lower fluid excretion by ultrafiltration and diuresis might be observed when using low-sodium solutions, for example containing between 102 and 120 mmol/l sodium, not compensated by glucose for reduced osmolarity. In the work of Rutkowski et al. peritoneal dialysis solutions with 125 mmol/l sodium were compared to standard-sodium solutions in hypertensive patients used in all daily exchanges (Rutkowski B, Tam P, van der Sande FM, et al. Low sodium versus standard-sodium peritoneal dialysis solution in hypertensive patients: a randomized controlled trial. Am J Kidney Dis. 2016;67(5):753-761). Although the noninferiority of the low-sodium PD solution for total Kt/Vᵤᵣₑₐ could not be proved, the study did show a beneficial clinical effects on sodium removal and blood pressure. However, a mild decrease in ultrafiltration, coherent with the reduced osmolarity, along with a modest decrease in urine output were observed. These disadvantages may become critical over longer period PD therapy particularly in hypertensive patients with poor control of volemia. A post-hoc analysis of the above clinical trial was conducted to dissect out the effect of the low-sodium solution on blood pressure depending on the residual kidney function (RRF) being higher or lower than 6 mL/min/1.73 m² [this is also known as glomerular filtration rate (GFR)] (Rutkowski B, Tam P, van der Sande FM, Vychytil A, Schwenger V, Klein G, Himmele R, Gauly A; Low Sodium balance Study Group. Residual Renal Function and Effect of Low-Sodium Solution on Blood Pressure in Peritoneal Dialysis Patients. Perit Dial Int. 2019; 39:335-343.). Only those patients with a glomerular filtration rate lower than 6 mL/min/1.73 m² and exposed for 12 weeks to a low-sodium containing PD solution showed a reduction of blood pressure, though episodes of hyponatremia were reported in this group of PD patients too.

Low-sodium peritoneal dialysis solutions are not yet used in clinical practice, in particular due to the disadvantages above described and of the risks of hyponatremia. Indeed, salt restriction, loop diuretics, icodextrin and judicious use of hypertonic glucose remain the options for hypertensive patients treated with peritoneal dialysis.

In view of the above, there is the need of a solution for peritoneal dialysis which can be used in hypertensive subjects, able to keep blood pressure under control and at the same time avoid undesired side-effects, such as decrease of ultrafiltration and hyponatremia.

US10251953 discloses a peritoneal dialysis solution having a sodium concentration between 121 and 129 mmol/l, in particular a solution with a sodium concentration of 125 mmol/l coupled with a chloride ion content of 91-94 mmol/l. A reduction of blood pressure was found and no apparent adverse events were reported. It is to be noted that this study has used the same sodium concentration used in the above mentioned Rutkowsky clinical trial but in the former study only 4 patients were treated for 4 weeks, whereas the latter was a randomized clinical trial with 40 patients per arm (control and experimental) and lasted 12 weeks. In addition and as discussed above, the Rutkowsky's clinical trial has shown some limitations.

US5589197 discloses peritoneal dialysis solutions comprising an osmotic agent, in particular dextrose, and sodium in a concentration lower than 132 mmol/l and having the same osmolality of peritoneal dialysis solutions with conventional sodium concentrations. However, only experimental results obtained with solutions having 120 mmol/l sodium are provided. It is not apparent why in this study a further reduction of the sodium content in peritoneal dialysis solution seemed to not affect ultrafiltration and diuresis as seen in the Rutkowsky clinical trial, where the extent of sodium reduction was significantly smaller (125 mM). Moreover, the low sodium peritoneal dialysis solution of the US5589197 was not compensated for the loss of osmolality due to the reduction of sodium concentration.

### SUMMARY OF THE INVENTION

It has now been found that the use of a solution for peritoneal dialysis comprising from 1.36 to 4.5% w/v of glucose and 130 mmol/l of sodium in hypertensive subjects is able to lower blood pressure without providing any side effect on ultrafiltration or other relevant parameters, such as residual diuresis and body weight.

Therefore, it is an object of the invention a solution for peritoneal dialysis comprising from 1.36 to 4.5% w/v of glucose and 130 mmol/l of sodium for use in the treatment of end-stage renal disease characterized in that the solution is used in hypertensive subjects, resistant or not resistant to anti-hypertensive therapy.

It has been found that the use of a solution with such a small reduction in sodium content (130 mEq/L), compared to standard peritoneal dialysis solutions available in commerce (132-134 mEq/L), is sufficient to reduce sodium overload over time, with consequent effect on blood pressure, advantageously avoiding deleterious effects on residual diuresis and ultrafiltration.

In hypertensive peritoneal dialysis subjects resistant to anti-hypertensive therapy, the use of such solution advantageously permits the achievement of normal blood pressure, despite the resistance to the anti-hypertensive therapy.

In hypertensive peritoneal dialysis subjects responding to anti-hypertensive therapy, the use of such solution advantageously allows the reduction or even elimination of the anti-hypertensive therapy, typically consisting in various drug combinations.

The solution and its uses and particular embodiments thereof will be described in the following.

### Detailed description of the invention

The manufacture of the solutions for peritoneal dialysis used in the present invention is well within the knowledge and abilities of the skilled in the art.

In the context of the present invention, for w/v% is intended weight/volume percentage concentration. This is a well-known way in the field to express the concentration of an ingredient in a solution.

In the solutions herein described, glucose is present in a concentration ranging from 1.36 to 4.5% w/v. Preferred glucose concentrations are 1.4, 1.5, 2.4, 4.0 % w/v.

Glucose can also be in hydrate form. In an embodiment, glucose is glucose monohydrate.

The solution further comprises sodium in the concentration of 130 mmol/l.

The solution can further comprise one or more ingredients such as electrolytes and buffers.

In particular, such further ingredient can be selected from the group consisting of: calcium, magnesium, chloride, lactate, potassium and bicarbonate.

Preferably, the solution further comprises:
- Calcium from 1.2 to 2.0 mmol/l, preferably 1.75 or 1.3 mmol/l;
- Magnesium from 0.20 to 0.75 mmol/l, preferably 0.5 mmol/l;
- Chloride from 90 to 120 mmol/l, preferably 98.6 or 101.5 mmol/l;
- Lactate from 25 to 45 mmol/l, preferably 35 mmol/l;
- Potassium from 0 to 4 mmol/l, preferably 2 mmol/l;
- Bicarbonate from 0 to 40 mmol/l.

The solution can have a pH comprised between 5.0 and 7.2.

The solution can contain further osmotic agents. Osmotic agent is a solute contained in a concentration sufficient to create an osmotic pressure to replace kidney function by diffusion from the patient's blood across the peritoneal membrane into the solution.

Osmotic agents for peritoneal dialysis are well-known in the art and preferably the agent is selected from the group consisting of: galactose; polyglucose; polyglycerol, fructose; sorbitol; amino acids, glycerol tripyruvate (tripyruvin), a single peptide, a mixture of peptides, polypeptides, pyruvate compound in the form of a sugar- pyruvate ester, a polyol-pyruvate ester, pyruvate thioester, a dihydroxyacetone-pyruvate ester and a dicarboxylic acid, in particular a C₂-C₁₅-dicarboxylic acid, such as dodecanedioic acid. Other osmotic agents, albeit not specifically mentioned here, are not excluded from the present invention.

Osmotic agents for use in peritoneal dialysis are commonly available and a description of these agents can be found in technical literature, for example WO01/26649, US5.126.373.

Conveniently, the solution according to the present invention, can further comprise a substance useful in the treatment of a patient in need of peritoneal dialysis. Such a substance will be determined by the person of ordinary skill in this art, depending on the needs of the patient and his or her conditions, such as the presence of other pathological states of particular needs. Examples of such substance are: vitamins, preferably soluble vitamins, antioxidants and antifibrotic agents (such as N-substituted 2-(1H) pyridone(s) and/or N- substituted 3-(1H) pyridones.

In an embodiment, the solution has the following composition:
Glucose 1.5% w/v
Sodium 130 mMol/l
Calcium 1.75 mMol/l,
Lactate 35 mMol/l,
Potassium 2 mMol/l
Magnesium 0.5 mMol/l

In an embodiment, the solution has the following composition:
Glucose 1.4% w/v,
Sodium 130 mMol/l
Calcium 1.3 mMol/l,
Lactate 35 mMol/l
Magnesium 0.5 mMol/l
Chloride 98.6 mMol/l

In an embodiment, the solution has the following composition:
Glucose 2.4% w/v,
Sodium 130 mMol/l
Calcium 1.3 mMol/l,
Lactate 35 mMol/l
Magnesium 0.5 mMol/l
Chloride 98.6 mMol/l

In an embodiment, the solution has the following composition:
Glucose 4%,
Sodium 130 mMol/l
Calcium 1.3 mMol/l,
Lactate 35 mMol/l
Magnesium 0.5 mMol/l
Chloride 98.6 mMol/l

The solutions herein described are usually contained, stored and delivered in a bag. The bag can be single-, dual- or multi-chamber. Such bags are of common use in the field of peritoneal dialysis and available on the market.

In an embodiment, the solutions herein described are manufactured in single-chamber bags. Such bags are typically used for containing acid pH solutions.

In another embodiment, the solutions herein described are manufactured in double-chamber bags, typically PVC-free. The double-chamber bag is preferably used for neutral pH solutions, which are typically better tolerated by the subjects when administered into the peritoneal cavity. Said double-chamber bag is typically a large format bag containing 1+1 litre of solution, divided by a "weak seal" that ensures sterilization, storage, and transport of the bags while avoiding contacts between the two solutions. Mixing of the two solutions present in Compartment A and in Compartment B is easily carried out by the patient simply pressing on the weak seal, obtaining the final solution to be used for PD treatment. Such bags are of common use in the field and available on the market.

Therefore, in an embodiment the solution used in the present invention is obtained by the mixing of two solutions stored in two compartments of a double-chamber bag.

In all the above embodiments of solutions prepared in a double-compartment bag (Compartment A and Compartment B), pH may be adjusted, for example with excipients as 25% hydrochloric acid or sodium hydroxide in Compartment A and sodium hydrogen carbonate in Compartment B. The skilled person knows how to perform these adjustments.

It has now been found that the solution herein described when used for peritoneal dialysis in hypertensive subjects affected by end-stage renal disease is able to lower blood pressure while at the same time not having the detrimental effects of the solutions of the prior art, in particular on ultrafiltration and sodium levels.

For hypertensive subject is intended a subject having a systolic and diastolic blood pressure above 120 and 80 mm Hg, respectively.

In an embodiment, such hypertensive subject is resistant to anti-hypertensive therapy. For subject resistant to anti-hypertensive therapy is intended a subject wherein the recommended treatment strategy fails to lower office systolic blood pressure (SBP) and diastolic blood pressure (DBP) values to <135 mmHg and/or <85 mmHg, respectively; usually the inadequate control of blood pressure is confirmed by ambulatory blood pressure monitoring (ABPM) or home blood pressure monitoring (HBPM) in patients whose adherence to therapy has been confirmed.

In an embodiment, such hypertensive subject is not resistant to anti-hypertensive therapy. This means that the blood pressure values of the subject are kept under control thanks to an anti-hypertensive therapy.

Anti-hypertensive therapy usually includes appropriate lifestyle measures, salt-restriction, and treatment with optimal or best-tolerated doses of three or more drugs, which typically include a diuretic, typically blockers of the renin-angiotensin system (angiotensin-converting enzyme inhibitors and angiotensin receptor blockers), calcium channel blockers, beta-blockers, alpha-blockers, centrally acting agents, and/or mineralocorticoid receptor antagonists.

The solution for the use of the invention can be used for continuous ambulatory peritoneal dialysis (CAPD) or for automated peritoneal dialysis (APD). Both are treatments well known in the field and the skilled person knows how to use the solutions herein described in those contexts.

The solution herein described can be used for nocturnal exchange and/or for one or more diurnal exchanges. The skilled person knows what is intended for nocturnal and diurnal exchanges.

For example, when the solution is used for nocturnal exchange one bag containing the solution is used by the subject for the nocturnal cycle. When the solution is used for diurnal exchanges, one or more bags containing the solution are used by the subject for daily cycles.

The peritoneal dialysis solution can be administered to the individual in need thereof once, twice, three or four times or more a day. In an embodiment, the solution herein disclosed is used for three diurnal exchanges. The solution herein disclosed can be used for all the nocturnal and/or diurnal exchanges or only for some of them, alternately with normal sodium solutions, such as peritoneal dialysis solutions containing sodium in the range of 132-134 mmol/l. In an embodiment, the solution herein disclosed is used for all the exchanges, in particular for all the diurnal exchanges.

The person skilled in the field, typically the nephrologist, is able to decide the number and kind of exchanges based on the conditions of the patient, the severity of the disease and the common general knowledge in the field.

The volume of the peritoneal dialysis solution administered intraperitoneally for each dose, or dwell, can be decided by the skilled person according to the general knowledge and the patients' need. In an exemplary embodiment, the volume of each dose or dwell is between about 1 to 3 liters, or between about 2 to 5 liters, preferably between about 1 to 3 liters for CAPD, and between about 2 to 5 liters for APD. For a pediatric patient the volume of the peritoneal dialysis solution administered is usually individualized according to intraperitoneal pressure measurements.

Typically, the peritoneal dialysis solution is left in the peritoneal cavity of the individual in need thereof for, or the dwell time is, between about 30 minutes up to 12 hours, or between about 1 hour and 10 hours.

The skilled person, such as a nephrologist, knows how to use the solutions for the herein disclosed uses in clinical practice according to general knowledge in the field. Reference can be made, for example to the Textbook of Peritoneal Dialysis, second edition, Gokal R,Khanna R, Krediet R, Nolph K (Editors). Kluwer Academic Publishers, 2000.

According to the present invention the solutions herein described are for use for the therapy of end-stage renal disease (ESRD).

End-stage renal disease (ESRD), also named kidney failure, is the last stage of chronic kidney disease, wherein dialysis is necessary.

The solution for peritoneal dialysis herein disclosed can be included in a package for peritoneal dialysis. A package for peritoneal dialysis is a set of supplies for executing one or more cycles of peritoneal dialysis. A package can contain, for example one or more bags containing the peritoneal dialysis solution of the present invention, even with different formulations, for example for day and/or night cycles. Additional supplies/equipment can be present, such as a disposable connector for executing the peritoneal dialysis cycle. Examples of packages are those commonly marketed.

The solutions herein disclosed can be delivered ready-to-use to the subject in need thereof or prepared at the time of use. In the latter circumstance, the solution can be prepared at home by the subject in need of peritoneal dialysis beginning with a source of purified water and adding the ingredients composing the solution, for example from one or more concentrates or powders, in order to obtain the fluid for peritoneal dialysis with the desired concentration of each ingredient. A peritoneal dialysis home machine can be used for this purpose. All such procedures and apparatus for self-preparing peritoneal dialysis solutions are known in the art, see for example Automated peritoneal dialysis with 'on-line'-prepared bicarbonate-buffered dialysate: technique and first clinical experiences (Brunkhorst R, Fromm S, Wrenger E, Berke A, Petersen R, Riede G, Westphale J, Zamore E, Ledebo I. Nephrol Dial Transplant. 1998 Dec;13(12):3189-92) and On-line preparation of solutions for dialysis: practical aspects versus safety and regulations (Ledebo I. J Am Soc Nephrol. 2002 Jan;13 Suppl 1:S78-83).

It is an object of the invention the solution for the use of the invention in the form of a concentrate or a powder, in particular to be used in a peritoneal dialysis home machine.

In an embodiment, the solution is in the form of a concentrate. Preferably, said concentrate comprises each of the osmotic ingredients in a concentration 2 to 10 fold the concentration of the osmotic agent in the final solution. For example, the osmotic agents may be provided as single bags containing the single osmotic ingredient at a concentration 2 to 10 fold its final concentration in the solution to be administered to the patient. A package comprising such bags for the uses herein disclosed is within the scope of the present invention. The concentrate solution may be present in sterile water or in sterile water containing salts and buffers present at a concentration ready to be administered to the patient. In another embodiment, a concentrated solution contains fix combination of the osmotic ingredients in a bag whereby their concentration is 2 to 10 fold of their final concentration in the solution to be administered to the patient. A package comprising such bag for the uses herein disclosed is within the scope of the present invention. The concentrate solution may be present in sterile water or in sterile water containing salts and buffers present at a concentration ready to be administered to the patient. The content of these concentrated bags will be diluted with locally prepared water with reverse osmosis, as it is done in hemodialysis done both at home or in dialysis centers, in order to obtain the final solution. The mixing and delivery of the final solution to the patient is done with a dialysis machine whose principle are well known by the experts in the field of peritoneal dialysis and hemodialysis.

In an embodiment, the solution is in a dry form, i.e. in the form of a powder. The osmotic ingredients either alone or according to the fix combinations of the various embodiments reported above can be prepared in a cartridge containing such ingredients in the dry form of a powder. A package comprising such cartridge for the uses herein disclosed is within the scope of the present invention. These cartridges also contain the necessary salts and buffer, as described in the various embodiments reported above. This kind of forms are of common use in the field of haemodialysis for the preparation of dialysis fluids. The content of the cartridges will be diluted with locally prepared water with reverse osmosis, as it is done in hemodialysis done both at home or in dialysis center, in order to obtain the final solution. The mixing and delivery of the final solution to the patient is done with a dialysis machine whose principle are well known by the experts in the field of peritoneal dialysis and hemodialysis.

The following examples further illustrate the invention.

### EXAMPLES

### Example 1

### Treatment of hypertensive subjects with low-sodium peritoneal dialysis solution

### Methods and results

Two hypertensive patients were treated with the low-sodium solution herein disclosed.

The first subject was an hypertensive patient responding to anti-hypertensive therapy.

Relevant data regarding such subject are as follows:
Chronic kidney failure from polycystic nephropathy and polycystic liver disease; systemic arterial hypertension; chronic obstructive pulmonary disease (COPD). The antihypertensive therapy consisted of a combination of an angiotensin receptor blocker and a calcium channel blocker.

On 16/02/2019, the patient started peritoneal dialysis treatment with three diurnal exchanges of a PD solution (Physioneal 35 - Baxter) containing 1,36% glucose and 132 mM sodium. The sodium concentration is the one commonly present in the Baxter PD solutions.

Self-measurement of blood pressure (BP) at home was within the normal range (see Table 1 below). In addition, on 12/06/2019, ambulatory BP monitoring (ABPM) was carried out (Table 2 below). ABPM devices are worn by patients over a 24-hour period with multiple measurements and are considered the gold standard for BP measurement (Little P, Barnett J, Barnsley L et al. Comparison of agreement between different measures of blood pressure in primary care and daytime ambulatory blood pressure. BMJ. 2002;325:254). It also has the advantage of measuring nocturnal BP and therefore allowing the detection of an attenuated dip during the night. As shown in Table 2, the 24 hrs average of systolic and diastolic BP were within the normal range, whereas the percentage of systolic and diastolic BP over the limits were 69.7% and 54.5%, respectively. No inversion of circadian rhythm (systolic night/day ratio: 0.88) was observed.

On 14/07/2019, the three diurnal exchanges with a PD solution containing 1,36% glucose and 132 mM sodium (Physioneal 35 - Baxter) were replaced with three diurnal exchanges of a PD solution (DextroCore LS - Iperboreal Pharma) containing 1,40% glucose where the sodium concentration was 130 mM instead of 132 Mm.

The composition of DextroCore LS (Iperboreal Pharma) was the following:
Sodio 130 mMol/l
Calcio 1,3 mMol/l
Magnesio 0,5 mMol/l
Lattato 35 mMol/l
Cloruro 98,6 mMol/l
Glucosio 77,78 mMol/l

Due to persistent low blood pressure values, on 27/08/2019 the antihypertensive therapy with calcium channel blocker was suspended.

On 08/01/2020, due to persistent low blood pressure values antihypertensive therapy with angiotensin receptor blocker was halved.

As described in the Table 1, after 8 months of treatment with a PD solution containing low sodium (130 mM), the dialytic prescription remained the same (three exchanges per day). In addition, compared to the beginning of the treatment with the low sodium PD solution, no change in body weight, ultrafiltration and residual diuresis were seen (Table 1). Home BP was also significantly reduced when compared to the values before the replacement with the low sodium PD solutions.

On 07/04/2020, a second ambulatory BP monitoring (ABPM) was carried out (Table 3). The 24 hrs average of systolic and diastolic BP were within the normal range, though significantly lower than the previous ABPM. Furthermore, the percentage of systolic and diastolic BP over the limits were 0.0% and 3.4%, respectively, values much lower than those observed in the previous ABPM. No inversion of circadian rhythm (systolic night/day ratio: 0.96) was observed. It is also worth noting that no significant change in body weight, ultrafiltration and residual diuresis was seen (Table 1).

On 08/04/2020 antihypertensive therapy with angiotensin receptor blocker was suspended too.

**Table 1. Description of the main clinical and laboratoristic features before the switch to 130-Na PD solution (DextroCore LS - Iperboreal Pharma) and after eight months of therapy with such solution.**

| | Before | After eight months |
|---|---|---|
| Creatinine (mg/dL) | 5.28 | 6.72 |
| Volume urine (mL/24 h) | 1700 | 1600 |
| Body weight (kg) | 69.0¹ | 70.3² |
| Ultrafiltration (mL/24h) | 450¹ | 485² |
| Serum sodium (mmol/L) | 138 | 139 |
| Serum Urea (mg/dL) | 144 | 151 |
| Home Systolic BP (mmHg) | 135¹ | 120² |
| Home Diastolic BP (mmHg) | 85¹ | 70² |

| | | |
|---|---|---|
| ¹Mean of all available measurements before the switch; ²Mean of all home measurements after the switch. Variability of body weight, daily ultrafiltration, urine volume, and home systolic and diastolic pressures was less than 8%. All BP measurements were performed by automatic sphygmomanometer. Body weight was performed by automatic scale. Ultrafiltration was calculated by difference in the weight of dialysate before and after each dwell. | | |

**Table 2. Ambulatory BP monitoring of the first patient on 12/06/2019**

| | | | | |
|---|---|---|---|---|
| Summary | | | | |

| Parameter | MED | STD | MIN | MAX |
|---|---|---|---|---|
| Systolic (mmHg) | 139 | 15.33 | 106 | 174 |
| Diastolic (mmHg) | 84 | 10.98 | 56 | 102 |
| Wrist pressure (mmHg) | 55 | 8.24 | 38 | 78 |
| Cardiac frequency (bpm) | 69 | 5.05 | 61 | 83 |

**Table 3. Ambulatory BP monitoring of the first patient on 07/04/2020**

| Summary | | | | |
|---|---|---|---|---|
| Parameter | MED | STD | MIN | MAX |
| Systolic (mmHg) | 115 | 7.40 | 98 | 134 |
| Diastolic (mmHg) | 69 | 7.46 | 57 | 90 |
| Wrist pressure (mmHg) | 46 | 7.62 | 24 | 65 |
| Cardiac frequency (bpm) | 75 | 6.87 | 55 | 87 |

The second subject was a hypertensive patient not responding to the anti-hypertensive therapy.

Relevant data regarding such subject are as follows:
Systemic arterial hypertension since about 10 years; under therapy for renal failure.

The antihypertensive therapy consisted of a combination of a diuretic, an alpha-blocker, an angiotensin receptor blocker and a calcium channel blocker.

On 17/06/2019, the patient started peritoneal dialysis treatment with three diurnal exchanges of a PD solution (Physioneal 35 - Baxter) containing 1,36% glucose and 132 mM sodium. The sodium concentration is the one commonly present in the Baxter PD solutions.

After 3 months of CAPD, home BP levels were still elevated (see Table 4, "before" column). To exclude pseudo-resistant hypertension [Borrelli S, De Nicola L, Stanzione G, Conte G, Minutolo R. Resistant hypertension in nondialysis chronic kidney disease. Int J Hypertens. 2013; 2013:929183.], we performed ABPM that showed a poor control of 24h (151/82 mmHg) diurnal (148/81 mmHg) and nocturnal BP (158/83 mmHg) with inversion of circadian rhythm (systolic night/day ratio: 1.07) (Table 5). The percentage of systolic and diastolic BP over the limits were 91.4% and 36.2%, respectively.

On 11/11/2019, the three diurnal exchanges with a PD solution (Physioneal 35 - Baxter) containing 1,36% glucose and 132 mM sodium were replaced with three diurnal exchanges of a PD solution (DextroCore LS - Iperboreal Pharma) containing 1,40% glucose where the sodium concentration was 130 mM instead of 132 mM.

The composition of DextroCore LS (Iperboreal Pharma) was the following:
Sodio 130 mMol/l
Calcio 1,3 mMol/l
Magnesio 0,5 mMol/l
Lattato 35 mMol/l
Cloruro 98,6 mMol/l
Glucosio 77,78 mMol/l

A significant reduction of home BP was evident after 1 month and this effect persisted for the following 4 months. As described in Table 4, no change in body weight, UF and residual diuresis was reported. Diet prescription was not changed. We then performed a second ABPM after six months reporting normal BP levels in 24h (131/73 mmHg), diurnal (133/76 mmHg) and nocturnal hours (125/65 mmHg) and improvement of circadian rhythm (systolic night/day ratio: 0.94) (Table 6). In addition, the percentage of systolic and diastolic BP over the limits were 50.0% and 6.5%, respectively, values much lower than those observed in the first ABPM.

**Table 4. Description of the main clinical and laboratoristic features before the switch to 130-Na PD solution (DextroCore LS - Iperboreal Pharma) and after five months of therapy with such solution.**

| | Before | After five months |
|---|---|---|
| Serum Creatinine (mg/dL) | 5.14 | 5.17 |
| Volume urine (mL/24 h) | 1300 | 1600 |
| Body weight (kg) | 56.0¹ | 56.7² |
| Ultrafiltration (mL/24h) | 330¹ | 365² |
| Serum sodium (mmol/L) | 137 | 137 |
| Serum Urea (mg/dL) | 173 | 177 |
| Home Systolic BP (mmHg) | 147¹ | 140² |
| Home Diastolic BP (mmHg) | 78¹ | 73² |

| | | |
|---|---|---|
| Mean of all available measurements before the switch; ²Mean of all home measurements after the switch. Variability of body weight, daily ultrafiltration, urine volume, and home systolic and diastolic pressures was less than 8%. All BP measurements were performed by automatic sphygmomanometer. Body weight was performed by automatic scale. Ultrafiltration was calculated by difference in the weight of dialysate before and after each dwell. | | |

**Table 5. Ambulatory BP monitoring of the second patient on 01/08/2019**

| Summary | | | | |
|---|---|---|---|---|
| Parameter | MED | STD | MIN | MAX |
| Systolic (mmHg) | 151 | 9.87 | 121 | 168 |
| Diastolic (mmHg) | 82 | 7.48 | 54 | 99 |
| Wrist pressure (mmHg) | 69 | 9.18 | 42 | 88 |
| Cardiac frequency (bpm) | 72 | 5.71 | 64 | 88 |

**Table 6. Ambulatory BP monitoring of the second patient on 15/04/2020**

| Parameter | MED | STD | MIN | MAX |
|---|---|---|---|---|
| Systolic (mmHg) | 131 | 11.77 | 108 | 166 |
| Diastolic (mmHg) | 73 | 9.47 | 50 | 103 |
| Wrist pressure (mmHg) | 58 | 10.55 | 30 | 78 |
| Cardiac frequency (bpm) | 71 | 6.44 | 56 | 86 |

### Conclusion

Our findings are consistent with Rutoksky et al. trial reporting that using uncompensated PD solutions with sodium concentration of 125 mEq/L was associated with a reduction of 9/5 mmHg in systolic and diastolic blood pressure (BP), respectively (Rutkowski B, Tam P, van der Sande FM, et al. Low sodium versus standard-sodium peritoneal dialysis solution in hypertensive patients: a randomized controlled trial. Am J Kidney Dis. 2016;67(5):753-761.). However, the administration of 125-sodium dialysate was associated with a significant reduction of urine volume and plasma sodium, as well as with no achievement of primary outcome of trial [Kt/V] compared to standard sodium group. Post-hoc analysis in PD patients with no residual diuresis showed in fact a higher effect on blood pressure levels associated with 125-sodium dialysate, suggesting a potential role of low sodium dialysate on hypertension in PD patients, not excluding a potential harmful effect on residual diuresis [Rutkowski B, Tam P, van der Sande FM, et al. Residual Renal Function and Effect of Low-Sodium Solution on Blood Pressure in Peritoneal Dialysis Patients. Perit Dial Int. 2019;39(4):335-343.].

For over 25 years it has been proposed that lowering dialysate sodium concentration could improve sodium overload. However, the use of ultra-low sodium (102-115 mEq/L) dialysate lead commonly to hyponatremia, hypotension and diuresis contraction, and, furthermore, these solutions needs to be compensated by hypertonic glucose solution to avoid ultrafiltration (UF) loss [Davies S, Carlsson O, Simonsen O, et al. The effects of low sodium peritoneal dialysis fluids on blood pressure, thirst and volume status. Nephrol Dial Transplant. 2009;24(5):1609-1617; Nakayama M, Yokoyama K, Kubo H, et al. The effect of ultra-low sodium dialysate in CAPD. A kinetic and clinical analysis. Clin Nephrol. 1996;45(3):188-193; Leypoldt JK, Charney DI, Cheung AK, Naprestek CL, Akin BH, Shockley TR. Ultrafiltration and solute kinetics using low sodium peritoneal dialysate. Kidney Int. 1995;48(6):1959-1966; Davies S, Haraldsson B, Vrtovsnik F, et al. Single-dwell treatment with a low-sodium solution in hypertensive peritoneal dialysis patients. Perit Dial Int. 2020; 40(5):446-454). However, the use of hypertonic glucose solutions is associated with peritoneal and systemic cardiovascular adverse effects.

Surprisingly, we found that a small reduction in sodium content (130 mEq/L), compared to standard PD solutions available in commerce (132-134 mEq/L) is sufficient to reduce sodium overload over time, with consequent effect on nocturnal blood pressure, avoiding deleterious effects on residual diuresis and ultrafiltration. Our low sodium PD solution not only permits the achievement of normal blood pressure in hypertensive PD patients resistant to anti-hypertensive therapy, but also in hypertensive PD patients responding to anti-hypertensive therapy along with the elimination of anti-hypertensive therapy often consisting in different drug combinations.

## Claims

1. A solution for peritoneal dialysis comprising from 1.36 to 4.5% w/v of glucose and 130 mmol/l of sodium for use in the treatment of end-stage renal disease **characterized in that** the solution is used in a hypertensive subject.

2. The solution for the use according to claim 1 wherein said hypertensive subject is resistant to anti-hypertensive therapy.

3. The solution for the use according to claim 1 wherein said hypertensive subject is not resistant to anti-hypertensive therapy.

4. The solution for the use according to anyone of the preceding claims, wherein said solution comprises glucose in a concentration selected from the group consisting of: 1.4 % w/v, 1.5 % w/v, 2.4 % w/v and 4.0 % w/v.

5. The solution for the use according to anyone of the preceding claims further comprising one or more ingredients selected from the group consisting of: calcium, magnesium, chloride, lactate, potassium and bicarbonate.

6. The solution for the use according to claim 5, which comprises:
- Calcium from 1.2 to 2.0 mmol/l, preferably 1.75 or 1.3 mmol/l;
- Magnesium from 0.20 to 0.75 mmol/l, preferably 0.5 mmol/l;
- Chloride from 90 to 120 mmol/l, preferably 98.6 or 101.5 mmol/l;
- Lactate from 25 to 45 mmol/l, preferably 35 mmol/l;
- Potassium from 0 to 4 mmol/l, preferably 2 mmol/l;
- Bicarbonate from 0 to 40 mmol/l.

7. The solution for the use of anyone of the preceding claims having a pH comprised between 5.0 and 7.2.

8. The solution for the use of anyone of the preceding claims having one of the following compositions:
(a)
Glucose 1.5% w/v
Sodium 130 mMol/l
Calcium 1.75 mMol/l,
Lactate 35 mMol/l,
Potassium 2 mMol/l
Magnesium 0.5 mMol/l;
(b)
Glucose 1.4% w/v,
Sodium 130 mMol/l
Calcium 1.3 mMol/l,
Lactate 35 mMol/l
Magnesium 0.5 mMol/l
Chloride 98.6 mMol/l;
(c)
Glucose 2.4% w/v,
Sodium 130 mMol/l
Calcium 1.3 mMol/l,
Lactate 35 mMol/l
Magnesium 0.5 mMol/l
Chloride 98.6 mMol/l;
or
(d)
Glucose 4%,
Sodium 130 mMol/l
Calcium 1.3 mMol/l,
Lactate 35 mMol/l
Magnesium 0.5 mMol/l
Chloride 98.6 mMol/l.

9. The solution for the use of anyone of the preceding claims wherein it is used for continuous ambulatory peritoneal dialysis (CAPD) or for automated peritoneal dialysis (APD).

10. The solution for the use of anyone of the preceding claims wherein it is used for one or more diurnal exchanges.

11. The solution for the use of claim 10 wherein it is used for three diurnal exchanges.

12. The solution for the use of anyone of the preceding claims, wherein the solution is contained, stored and/or delivered in a bag, such as a single-, dual-or multi-chamber bag.

13. The solution for the use of anyone of the preceding claims, wherein said solution is in the form of a concentrate or in dry form.

14. The solution for the use of claim 13, for use in a peritoneal dialysis home machine.
